# EUROPEAN PATENT SPECIFICATION

(11) **EP 1 711 268 B1**
(45) Date of publication and mention of the grant of the patent: **02.05.2007**
(21) Application number: 05722615.1
(22) Date of filing: 02.02.2005
(51) Int. Cl.: B04B 5/04, G01N 21/07

(54) **USER-CONFIGURABLE ANALYTICAL ROTOR SYSTEM**
BENUTZERKONFIGURIERBARES ANALYTISCHES ROTORSYSTEM
SYSTEME A ROTOR ANALYTIQUE POUVANT ETRE CONFIGURE PAR L'UTILISATEUR

(30) Priority: 04.02.2004 US 541623 P; 16.11.2004 US 989581; 16.11.2004 US 989473; 16.11.2004 US 989474; 16.11.2004 US 989475; 16.11.2004 US 989570
(43) Date of publication of application: 18.10.2006
(73) Proprietor: Hach Company, Loveland, CO 80539 (US)
(72) Inventor: MOORE, Leon, E., Windsor, CO 80550 (US)
(74) Representative: Bibby, William Mark
(86) International application number: PCT/US2005/002811
(87) International publication number: WO 2005/077541

(56) References cited:
- FR-A- 2 537 281
- US-A- 3 798 459
- US-A- 4 147 294
- US-A- 5 457 053
- US-B1- 6 348 176

## Description

### Background of the Invention

### 1. Field of the Invention

The invention is related to the field of analytical rotors, and in particular, to analytical rotors that allow the user to select the individual blocks that comprise the analytical rotor.

### 2. Statement of the Problem

An analytical rotor system performs a test on a sample. The test could be the detection of an analyte, such as the detection of a dissolved metal in a fresh water sample. The analytical rotor system includes a plastic disc-shaped rotor. The disc-shaped rotor includes a sample chamber in the center and capillaries and chambers that extend from the sample chamber towards the edge of the rotor.

To perform the test, the sample is placed in the sample chamber in the center of the rotor, and the system spins the rotor to create centrifugal force. The centrifugal force transfers the sample from the central sample chamber through a capillary to a chamber that typically contains a reagent to interact with the sample. The spin may be accelerated, decelerated, stopped, and reversed to control sample flow through the rotor. Capillary action also draws the sample through the rotor. Thus, a combination of centrifugal force and capillary action transfers a precise amount of the sample to specific locations in the rotor for specific amounts of time.

In a typical test, the sample is transferred from the central sample chamber to a reagent chamber that contains a reagent to interact with the sample. After the interaction, the sample is then transferred from the reagent chamber to an analytical chamber. A system transmitter transfers an analytical signal through the sample in the analytical chamber to a receiver. The test is completed by analyzing the received analytical signal.

Current rotors are pre-configured for a single test or set of tests. This condition leads to a series of problems for users and suppliers alike.

From the user's perspective, the user must locate and purchase a rotor that is pre-configured for the set of tests that they desire. In many cases, the user cannot locate a single rotor that can perform the entire set of tests that they desire. The user must then purchase multiple rotors. The need to purchase multiple rotors can increase the cost of the tests, especially when the multiple rotors include extra functionality that is paid for but not used.

In addition, the use of multiple rotors adds unwanted complexity to the testing process. If three rotors are required to complete a desired set of tests, the first rotor is mounted on the analytical device, loaded with a sample, and spun to perform some of the tests. The first rotor is then removed from the analytical device, and the second rotor is mounted on the analytical device, loaded with the sample, and spun to perform some of the tests. The second rotor is then removed from the analytical device, and the third rotor is mounted on the analytical device, loaded with the sample, and spun to perform the rest of the tests.

More time is required to use the three rotors in sequence than would be required if a single rotor were available to perform all of the tests. In addition to the increased testing time, the sample is handled multiple times to load each rotor. The repeated handling of the sample increases the risk of sample contamination and waste. The repeated loading of the sample may require more sample than is available.

From the supplier's perspective, the supplier should minimize the undesirable need for multiple rotors. Thus, the supplier must anticipate the tests that users desire in a single rotor. If the supplier is wrong, then money and time are wasted to pre-configure a rotor that nobody wants. To offer a robust selection of different rotors that each perform a different set of tests, the supplier would have to maintain a rather large rotor inventory. Large inventories are expensive and undesirable for the supplier.

Thus, current analytical rotor systems do not readily support unique or custom combinations of tests without designing and manufacturing unique and customized rotors. This situation causes problems for both the suppliers and the users of such systems.

Current analytical rotor systems exhibit other problems. For example, some current analytical rotor systems have analytical chambers where an analytical signal passes through a processed sample. The analytical signal is then processed to characterize the sample. The size and orientation of the analytical chamber defines a distance that the analytical signal passes through the sample - referred to as the analytical signal path. Current analytical rotor systems do not have long enough analytical signal paths to properly perform some tests, such as tests for low concentrations of analytes. Thus, the small size of current analytical signal paths prevents or inhibits rotor systems from performing such tests.

In addition, the central sample chamber that initially holds the sample and transfers the sample to the rotor may allow the sample to leak while the system is not spinning. Also, rotor technology has not been effectively applied to perform some tests in an automated fashion.

US 634 8176 B1 discloses a "cartridge-based" instrument that is designed to receive and process individual self-contained cartridges which are manually pre-loaded with sample and any required reagents.

### Summary of the Solution

In accordance with the present invention an analytical rotor system is provided that is configured to perform a plurality of tests selected by a user on at least one sample. The analytical rotor comprises a plurality of rotor blocks and a rotor base. The rotor blocks are each configured to perform at least one of the tests on the at least one sample in response to centrifugal force. The rotor blocks are physically separate units from one another. The rotor base is a physically separate unit from the rotor blocks and is configured to allow the user to manually install the rotor blocks on the rotor base. The rotor base is configured to hold the installed rotor blocks in place during the centrifugal force and to connect to an analytical device that provides the centrifugal force. The rotor base includes a sample chamber configured to receive and hold the sample, and in response to the centrifugal force, to transfer the sample through a sample port to one of the rotor blocks.

In some examples of the invention, the rotor base includes flanges configured to secure the rotor blocks to the rotor base during the centrifugal force.

In some examples of the invention, one of the tests is to determine a concentration of an analyte in the at least one sample and the at least one sample comprises a water sample. In some examples of the invention the sample chamber is tapered so a fluid level of the sample does not reach the sample port while the sample chamber is at rest, but the fluid level of the sample does reach the sample port when the sample chamber is spinning.

In some examples of the invention, one of the rotor blocks is configured to filter the at least one sample.

In some examples of the invention, one of the tests comprises a titration test.

In some examples of the invention, at least one of the rotor blocks includes a plurality of titration chambers that hold different proportions of a titration reagent and the sample in response to the centrifugal force and wherein at least one of the titration chambers indicates an event that corresponds to one of the proportions of the sample and the titration reagent.

In some examples of the invention, one of the tests comprises a method of standard additions test.

In some examples of the invention, at least one of the rotor blocks includes a sample-only chamber and a standard addition chamber, wherein the sample only chamber is configured to hold a first portion of the sample in response to the centrifugal force but does not hold a standard, and wherein the standard addition chamber is configured to hold a first portion of the standard that is added to a second portion of the sample in response to the centrifugal force.

In some examples of the invention, one of the rotor blocks includes an analytical chamber that is configured to allow an analytical signal to traverse an analytical signal path through the analytical chamber to perform one of the tests, wherein the analytical signal path is parallel to a plane of a spin that provides the centrifugal force.

In accordance with the invention a method of operating an analytical rotor system is provided to perform a plurality of tests selected by a user on at least one sample. The method is characterized by: holding the at least one sample in a sample chamber in a rotor base manually installing a plurality of rotor blocks on the rotor base, wherein the rotor blocks are physically separate units from one another, and wherein the rotor base is a physically separate unit from the rotor blocks; connecting the rotor base to an analytical device; and operating the analytical device to spin the rotor base to provide centrifugal force that causes each of the rotor blocks to perform at least one of the tests on the at least one sample, wherein the rotor base is configured to hold the installed rotor blocks in place during the centrifugal force, and wherein operating the analytical device to spin the rotor base to provide the centrifugal force comprises transferring the sample through a sample port to one of the rotor blocks in response to the centrifugal force.

In some examples of the invention, the rotor base includes flanges configured to secure the rotor blocks to the rotor base during the centrifugal force.

In some examples of the invention, one of the tests is to determine a concentration of an analyte in the at least one sample and the at least one sample comprises a water sample.

In some examples of the invention, the sample chamber is tapered so a fluid level of the sample does not reach the sample port while the sample chamber is at rest, but the fluid level of the sample does reach the sample port when the sample chamber is spinning.

In some examples of the invention, one of the rotor blocks filters the at least one sample to perform one of the tests.

In some examples of the invention, one of the tests comprises a titration test.

In some examples of the invention, at least one of the rotor blocks includes a plurality of titration chambers that hold different proportions of a titration reagent and the sample in response to the centrifugal force, and wherein to perform the at least one test, at least one of the titration chambers indicates an event that corresponds to one of the proportions of the sample and the titration reagent.

In some examples of the invention, one of the tests comprises a method of standard additions test.

In some examples of the invention, at least one of the rotor blocks includes a sample-only chamber and a standard addition chamber, and wherein to perform the at least one test, the sample only chamber holds a first portion of the sample in response to the centrifugal force but does not hold a standard, and wherein the standard addition chamber holds a first portion of the standard that is added to a second portion of the sample in response to the centrifugal force.

In some examples of the invention, one of the rotor blocks includes an analytical chamber that is configured to allow an analytical signal to traverse an analytical signal path through the analytical chamber to perform one of the tests, wherein the analytical signal path is parallel to a plane of a spin that provides the centrifugal force.

### Description of the Drawings

Figure 1 illustrates a perspective view of a rotor base and a rotor block for an analytical rotor system in an example of the invention.
Figure 2 illustrates a top view of an analytical rotor system in an example of the invention.
Figure 3 illustrates a rotor block for an analytical rotor system in an example of the invention.
Figure 4 illustrates a set of rotor blocks for an analytical rotor system in an example of the invention.
Figure 5 illustrates a rotor block for an analytical rotor system in an example of the invention.
Figure 6 illustrates a rotor base sample chamber for an analytical rotor system in an example of the invention.
Figure 7 illustrates a rotor base sample chamber for an analytical rotor system in an example of the invention.
Figure 8 illustrates a rotor block to perform a titration for an analytical rotor system in an example of the invention.
Figure 9 illustrates a rotor block to perform a method of standard additions for an analytical rotor system in an example of the invention.

### Detailed Description of the Invention

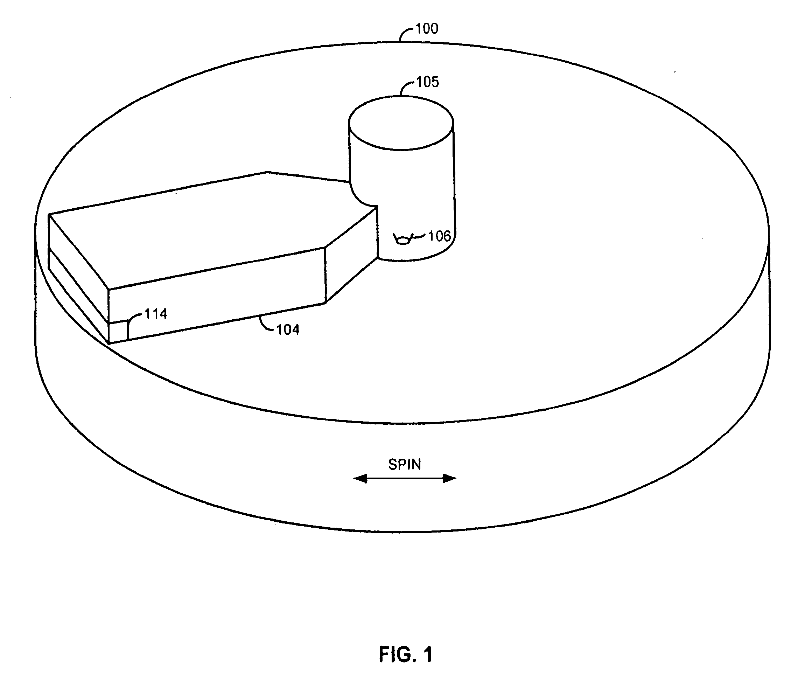

FIGS. 1-9 and the following description and Exhibits depict specific examples to teach those skilled in the art how to make and use the best mode of the invention. For the purpose of teaching inventive principles, some conventional aspects have been simplified or omitted. Those skilled in the art will appreciate variations from these examples that fall within the scope of the invention. Those skilled in the art will appreciate that the features described below can be combined in various ways to form multiple variations of the invention. As a result, the invention is not limited to the specific examples described below, but only by the claims and their equivalents.

### Analytical Rotor System with Modular Rotor Blocks

Figure 1 illustrates a perspective view of rotor base 100 and rotor block 104 in an example of the invention. Rotor base 100 includes sample chamber 105 and flange 114. Sample chamber 105 includes sample port 106 that protrudes from sample chamber 105. Rotor base 100 and rotor block 104 may be comprised of clear plastic.

Rotor base 100 and rotor block 104 are physically separate units. The user selects and manually installs rotor block 104 on rotor base 100. Rotor block 104 is configured to perform a test or set of tests when a sample is loaded into sample chamber 105 and rotor base 100 spins. The user would typically select and install additional rotor blocks on rotor base 100 to perform additional tests at the same time, but the additional blocks are not shown on Figure 1 for clarity.

When installed, rotor block 104 couples to a sample port on sample chamber 105 (this sample port is not shown but is like port 106). The sample port protrudes from sample chamber 105 through an orifice in rotor block 104. Flange 114 engages the back and sides of rotor block 104 at the edge of rotor base 100. Together, flange 114 and the sample port on sample chamber 105 provide a physical interface that secures rotor block 104 to rotor base 100 when rotor base 100 spins, but that also permits easy manual installation and de-installation of rotor block 104 by the user.

When rotor base 100 spins, it is important to prevent rotor block from sliding off of base 100. Flange 114 prevents such sliding. In addition, flange 114 prevents rotor block from sliding from side-to-side as the spin stops, starts, or reverses. It is also important to prevent rotor block 104 from tipping upward at the center of block 100 and flying off of base 100. The protruding sample port (not shown) on sample chamber 105 fits into an orifice on rotor block 104 to prevent such tipping.

Various alternative physical interfaces could also be used to secure rotor block 104 on spinning base 100, while allowing easy manual installation by the user. Such alternates include posts extending from base 100 and corresponding holes in the bottom of block 104 or posts protruding from the bottom of block 104 and corresponding holes in block 100. Instead of a male port on sample chamber 105 and an orifice on block 104, sample chamber 105 could have the orifice, and block 104 could have the male port. Other physical interfaces that are suitable for securing block 104 to spinning base 100 include adhesive surfaces, Velcro, snaps, and straps. Those skilled in the art will appreciate other physical interfaces that are suitable for securing block 104 to spinning base 100, but that also allow for easy manual installation and de-installation of block 104 by the user.

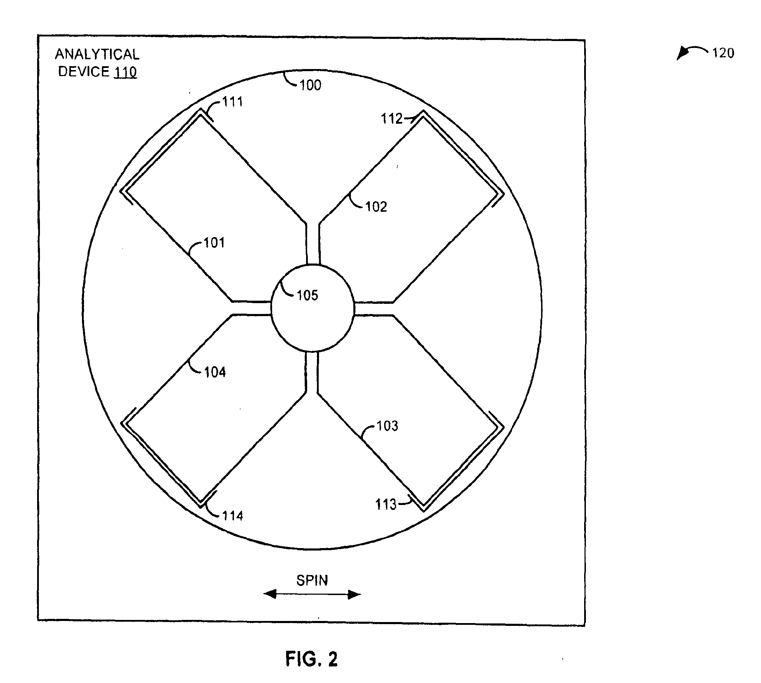

Figure 2 illustrates a top view of analytical rotor system 120 in an example of the invention. Analytical rotor system 120 includes analytical device 110, rotor base 100, and rotor blocks 101-104. Rotor blocks 101-104 are each configured to perform a test or set of tests. Thus, the user selects rotor blocks 101-104 to perform the tests desired by the user, and manually installs the selected blocks on base 100. The tests may be the repeated versions of the same test or may be different tests. The tests may be performed on different samples or different portions of the same sample.

Rotor base 100 includes sample chamber 105 and raised flanges 111-114. Rotor base 100 may include a physical interface that is similar to that used by conventional rotors for attachment to analytical device 110. Thus, rotor base 100 may be manually installed on analytical device 110 in the same manner that a conventional disc-shaped rotor is manually installed on a conventional analytical device. For example, base 100 may have a pin the fits within a socket on analytical device 110. Flanges 111-114 and protruding sample ports (not shown) secure rotors 101-104 to rotor base 100.

In operation, the user first selects the tests to perform. The user then selects the necessary rotor blocks (blocks 101-104 in this example) to perform the selected tests. The user then installs selected rotor blocks 101-104 on rotor base 100 and installs rotor base 100 on analytical device 110. The user also loads the sample into sample chamber 105. The user operates analytical device 110 to spin rotor base 100 to generate centrifugal force. The centrifugal force drives the sample from sample chamber 105 into rotor blocks 101-104. The centrifugal force and capillary action force the sample through rotor blocks 101-104 to perform the various tests.

Analytical device 110 carefully controls the spin of base 100. This control is implemented through a spin profile that specifies when the base spins and when the base is still. For a given spin, the profile specifies the direction, speed, acceleration, deceleration, and duration of the spin. The spin control directs the propagation of the sample through the rotor blocks, including the precise amount of sample that is transferred and how long a transferred sample interacts with a reagent. Those skilled in the art are familiar with spin profiles and spin control.

Analytical device 110 may use spectrophotometry, fluorescence, electrochemistry, titration, visual detection, kinetic assays, method of standard additions, and/or some other technique to test the sample. Those skilled in the art could adapt conventional analytical devices based on this disclosure to develop analytical device 110. Abaxis, Inc. of California supplies such analytical devices.

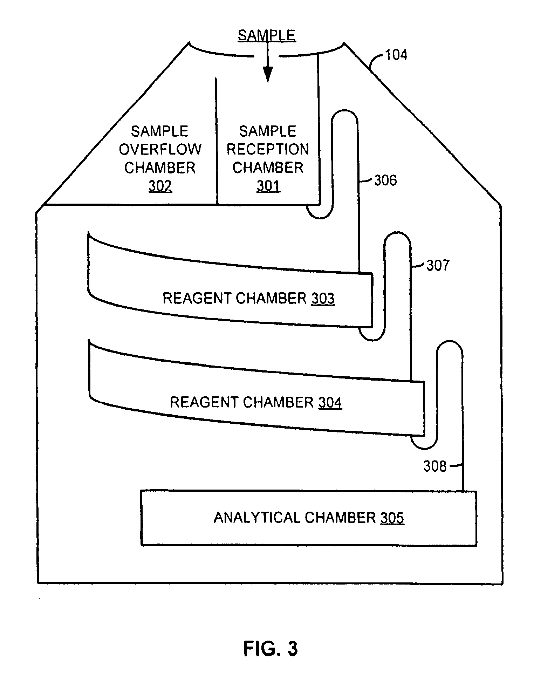

Figure 3 illustrates rotor block 104 in an example of the invention. Rotor block 104 includes sample reception chamber 301, sample overflow chamber 302, reagent chambers 303 and 304, analytical chamber 305, and capillaries 306-308. Rotor block 104 also contains vents that are not shown for clarity.

In operation, centrifugal force generated by a spinning rotor base (not shown) drives the sample into sample reception chamber 301. Excess sample overflows into sample overflow chamber 302. The overflow mechanism loads sample reception chamber 301 with a precise amount of the sample. Using a combination of capillary action and centrifugal force, a precise amount of the sample in sample reception chamber 301 is delivered by capillary 306 to reagent chamber 303. Reagent chamber 303 contains a reagent that interacts with the sample. After the desired interaction, capillary action and centrifugal force transfer a precise amount of the reacted sample in reagent chamber 303 through capillary 307 to reagent chamber 304. Reagent chamber 304 also contains a reagent that interacts with the sample. After the desired interaction, capillary action and centrifugal force transfer a precise amount of the reacted sample in reagent chamber 304 through capillary 308 to analytical chamber 305. The analytical device (not shown) may include a transmitter and receiver to transmit an analytical signal through analytical chamber 305 and receive the analytical signal after it passes through the reacted sample in analytical chamber 305. Analytical device 110 processes the received analytical signal to complete the test.

Other rotor block designs could also be used. Typically, rotor blocks would come in various different designs to support various different tests. Some rotor blocks may have no reagent chambers while other blocks may have multiple reagent chambers. Some rotor blocks may have chambers for buffers or diluents.

On Figure 3, chambers 303-305 and capillaries 306-308 form a process path from sample reception chamber 301 to the outer edge of block 104. A rotor block may have multiple parallel process paths from sample reception chamber 301 to the outer edge of block 104. These parallel paths may placed side-by-side or they may be stacked on top of one another. Multiple paths may merge together, or a single path could diverge into multiple paths.

The design and manufacture of conventional disc-shaped rotors with chambers, capillaries, and vents to process a sample in the presence of centrifugal force is well known in the art. The same general techniques can be used to implement chambers, capillaries, and vents within the rotor blocks to process a sample in the presence of centrifugal force. One difference between modular rotor blocks and conventional disc-shaped rotors is that a conventional rotor is a single physically integrated unit, but a modular set of rotor blocks are not physically integrated together. Advantageously, the user may select and install the modular rotor blocks to easily customize their own analytical rotor.

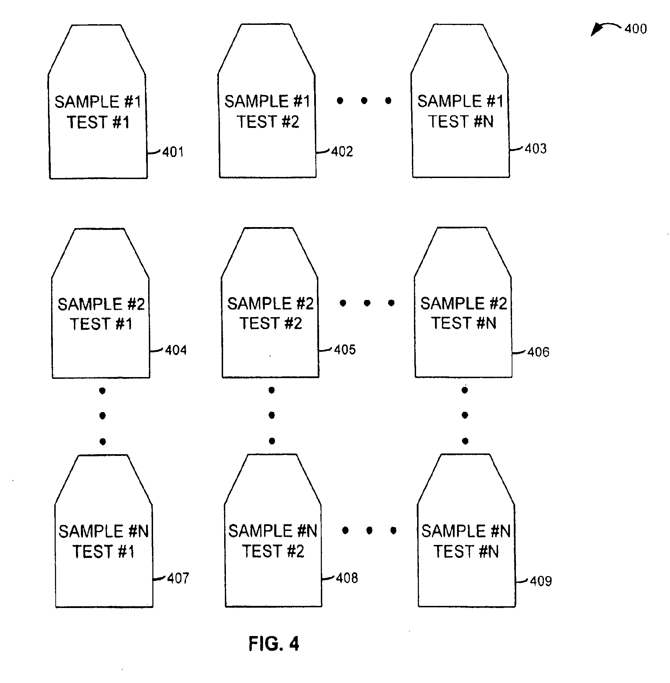

Figure 4 illustrates a set 400 of rotor blocks 401-409 for an analytical rotor system in an example of the invention. Each rotor block is physically separate from the other rotor blocks. Thus, each rotor block is a discreet unit that may be selected and used independently of the other rotor blocks that may be selected and used. Rotor blocks 401-409 could be similar to rotor block 104 or could use some other design variation.

Rotor block 401 is configured to perform test #1 on sample #1. Rotor block 402 is configured to perform test #2 on sample #1. Rotor block 403 is configured to perform test #N on sample #1. Thus, rotor blocks 401-403 perform three different tests on sample #1. Likewise, rotor block 404 is configured to perform test #1 on sample #2. Rotor block 405 is configured to perform test #2 on sample #2. Rotor block 406 is configured to perform test #N on sample #2. Thus, rotor blocks 404-406 provide multiple blocks for different tests on sample #2. Likewise, rotor block 407 is configured to perform test #1 on sample #N. Rotor block 408 is configured to perform test #2 on sample #N. Rotor block 409 is configured to perform test #N on sample #N. Thus, rotor blocks 407-409 provide multiple blocks for different tests on sample #N.

It should be appreciated that set 400 provides a robust group of rotor blocks for user-selection based on the samples and tests of interest to the user. The various tests may be as simple as placing a specific amount of the sample in the analytical chamber without any reagent interaction, but the tests may also be relatively complex involving multiple reagent interactions with various reagents.

The statement that a rotor block performs a test does not mean that the rotor block performs the entire test by itself. The rotor block typically requires the base and analytical device to generate the centrifugal force, provide the sample, and possibly to transmit, receive, and process an analytical signal. In the context of the invention, a rotor block performs a test by performing at least a part of the test. Other components may perform other parts of the test as well.

One example of a test is to determine the concentration of an analyte in a sample. Examples of these analytes include manganese, iron, nitrate/nitrite, and copper or some other substance. One example of a sample is a water sample, such as drinking water, fresh water, and sea water. Other tests include aliquating, enzyme-based tests, method of standard additions, and filtration. One example of an enzyme-based test is an Enzyme-Linked Immuno-Sorbent Assay (ELISA).

Consider a situation where the user desires to test a water sample for concentrations of manganese, iron, nitrate/nitrite, and copper. In prior systems, the user would have had to locate a pre-configured disc-shaped rotor that handles all of these tests or purchase multiple such rotors and perform repeated tests. In contrast, the present system would allow the user to select a first rotor block that tests for manganese detection, a second rotor block that tests for iron detection, a third rotor block that tests for nitrate/nitrite detection, and a fourth rotor block that tests for copper detection. The user would easily install the selected rotor blocks on the rotor base, and perform all tests in a single pass without having to reload the sample or change rotors.

### Analytical Chambers with Longer Analytical Signal Paths

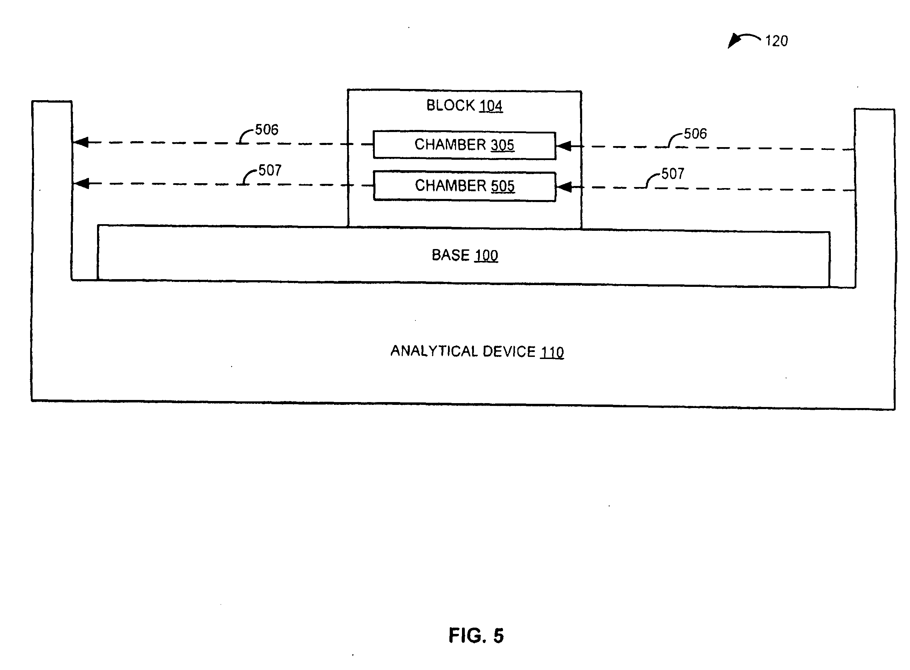

Figure 5 illustrates analytical rotor system 120 in an example of the invention. Analytical rotor system 120 includes rotor block 104 that is mounted on rotor base 100, which is mounted on analytical device 110. The view on figure 5 is looking from the outside edge toward the center of base 100 and into the end of rotor block 104. Flange 114 is omitted from figure 5 for clarity. On figure 5, rotor block 104 includes analytical chambers 305 and 505.

In operation, analytical device 110 spins base 100 to process a sample, and eventually, the processed sample is loaded into analytical chamber 305. Note that sample processing may include multiple reagent interactions or may simply load analytical chamber 305 with the appropriate amount of sample from sample reception chamber 301. To test the sample in analytical chamber 305, analytical device 110 spins base 100 to properly position analytical chamber 305 in line with the path of analytical signal 506. Analytical device 110 then transfers analytical signal 506 through analytical chamber 305 where analytical signal 506 interacts with the sample. Analytical device 110 receives analytical signal 506 after it passes through analytical chamber 305. Note that flange 114 (see FIGS. 1-2) should be configured to avoid blocking analytical signal 506 as it enters and exits block 104. Analytical device 110 processes received analytical signal 506 to finish the test. For example, analytical device 110 may process analytical signal 506 to determine the concentration of an analyte in the sample.

The distance that analytical signal 506 traverses analytical chamber 305 is referred to as the analytical signal path. Note that this analytical signal path is parallel to base 100 and the spin plane, which are horizontal on Figure 5. The length of the analytical signal path can be increased by widening block 104 and analytical chamber 305. During the design phase, the length of the analytical signal path may be lengthened to support the desired test for the rotor block. For example, possible analytical signal path lengths could start at 1/16 of an inch with additional signal paths at 1/16 inch increments up to a total length of six inches.

In prior systems, the orientation of the analytical signal path was perpendicular to base 100 and the spin plane. Thus, prior analytical signal paths are vertically oriented with the analytical signal having vertical propagation. This prior signal path could only be increased by increasing the height of the rotor - which has severe practical limitations because of size constraints, such as shipping and storage costs.

Since the prior vertical analytical signal path is restricted in size, prior rotors are not suitable to determine a low concentration of certain analytes in the sample, because the analytical signal is not exposed to enough of the sample to detect the low concentration. Advantageously, the longer analytical signal path on figure 5 exposes enough sample to analytical signal 506 to allow the analytical device 110 to detect a low concentration of an analyte in the sample.

In addition, block 104 includes analytical chamber 505 directly below and parallel to analytical chamber 305. To test a sample in analytical chamber 505, analytical device 110 transfers analytical signal 507 through analytical chamber 505, where analytical signal 507 interacts with the sample. Analytical device 110 receives analytical signal 507 after it passes through analytical chamber 505. Analytical device 110 processes received analytical signal 507 to finish the test. Note that the analytical signal path for chamber 505 is also parallel to base 100 and the spin plane, and thus, provides the same benefits discussed above for chamber 305.

In some cases, centrifugal force and capillary action transfer some of the sample from sample overflow chamber 302 (See Figure 3) to analytical chamber 505. Thus, an unprocessed portion of the sample can be placed in chamber 505 while a processed portion of the sample is placed in chamber 305. Advantageously, the analysis of both processed and unprocessed samples may be carried out as described above for comparative purposes. Alternatively, chamber 505 may be loaded with a processed sample like chamber 305, instead of loading the unprocessed sample.

Note that figure 5 shows block 104 as having two levels - an upper level having chamber 305 and a lower level having chamber 505. Each level could have its own chambers and capillaries to support two separate tests on the sample. In addition, sample reception chamber 301 could be placed in the upper level, and sample overflow chamber 302 could be placed in the lower level below sample reception chamber 301. The analytical signal path is described above with respect to a modular rotor block, but in some examples of the invention, the analytical signal path could also be implemented in an otherwise conventional disc-shaped rotor

### Rotor Base Sample Chambers

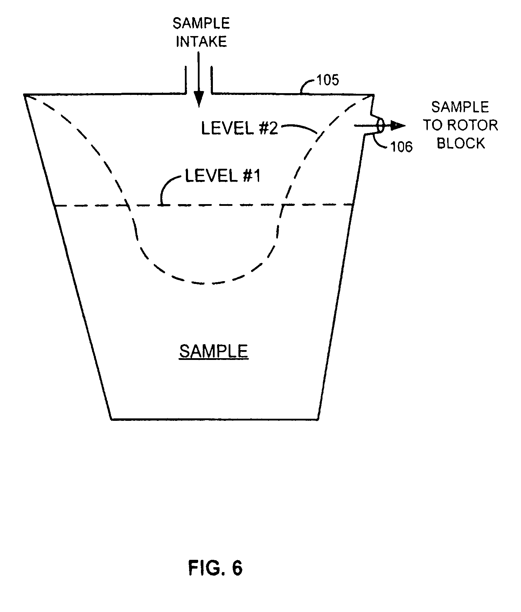

Figure 6 illustrates sample chamber 105 in an example of the invention. Sample chamber 105 includes sample port 106, and sample chamber 105 typically includes other similar ports that are not shown for clarity. Sample chamber 105 is tapered, so the bottom is narrower than the top. Note that sample port 106 is located substantially at the top of sample chamber 105. Sample chamber 105 has an upper barrier with a sample intake port where the user may load the sample into chamber 105.

While analytical device 110 is not operating, sample chamber 105 is at rest, and the loaded sample rests at fluid level #1. As analytical device 110 operates, sample chamber 105 spins, and the centrifugal force drives the sample to fluid level #2, where the sample egresses through sample port 106 to the rotor block. Advantageously, when sample chamber 105 is at rest and the sample is at fluid level #1, the sample cannot reach sample port 106. The sample is provided to sample port 106 at fluid level #2 only when the system is operating and sample chamber 105 spins. Thus, sample chamber 105 inhibits sample leakage through sample port 106 while analytical device 110 is not operating.

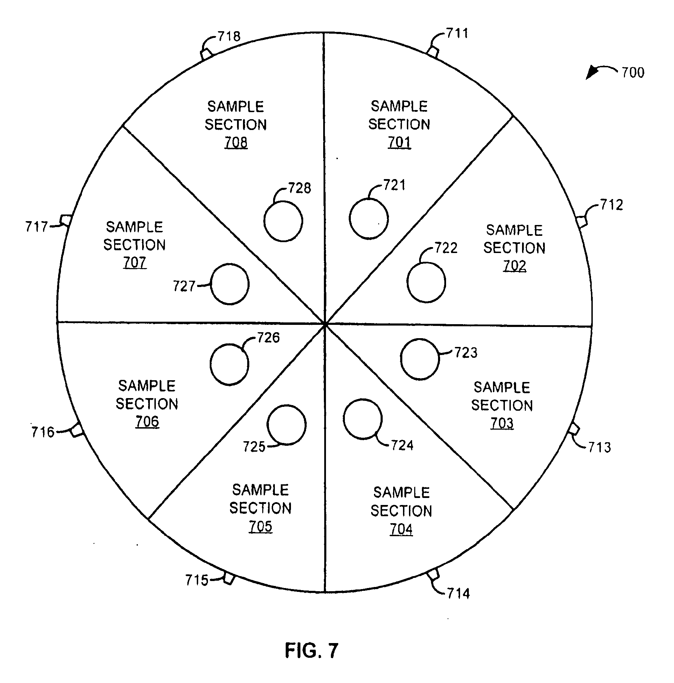

Figure 7 illustrates sample chamber 700 in an example of the invention. Sample chamber 700 could be integrated onto base 100 as an alternative to sample chamber 105. Sample chamber 700 is separated into sample sections 701-708. Sample sections 701-708 have respective sample ports 711-718. The sample ports couple to respective rotor blocks when the rotor blocks are installed on base 100. Sample sections 701-708 also have respective sample intakes 721-728. Sample sections 711-708 are each configured to receive and dispense its own sample.

In operation, the user selects the tests and samples of interest, and obtains the corresponding rotor blocks for the selected tests and samples. The user loads the samples into samples sections 701-708 and installs the selected rotor blocks to the appropriate sample ports 711-718. When sample chamber 105 spins, sample chambers 701-708 dispense the samples to their respective rotor blocks through sample ports 711-718.

Advantageously, sample chamber 700 facilitates the simultaneous testing of multiple samples with multiple rotor blocks. For example, water samples from eight different locations may be taken and loaded into sample sections 701-708. Eight rotor blocks could be loaded onto base 100, where each rotor block is designed to determine the concentration of a metal in water. With a single test, the concentration of metal in water samples from eight different locations can be obtained. Eight sample sections are shown on Figure 6, but the number could be increased or decreased as desired. In addition, each sample section could incorporate the tapered design and port location of Figure 6 to inhibit sample leakage when sample chamber 700 is at rest.

In the examples of FIGS. 6-7, sample chambers 105 and 700 may be pre-loaded with a substance to interact with the sample prior to transfer to the rotor block. The substance could perform oxidation, acid digestion, pH/ ionic strength adjustment, precipitation, or some other operation on the sample. The substances could include a buffer, a masking agent, or some other treatment for the sample. Since these substances may corrode plastic, sample chambers 105 and 700 may be internally lined with glass, ceramic, or some other non-corrosive material. The sample chamber is described above with respect to a modular rotor block, but in some examples of the invention, the sample chamber could also be implemented in an otherwise conventional disc-shaped rotor

### Titration Rotor Block

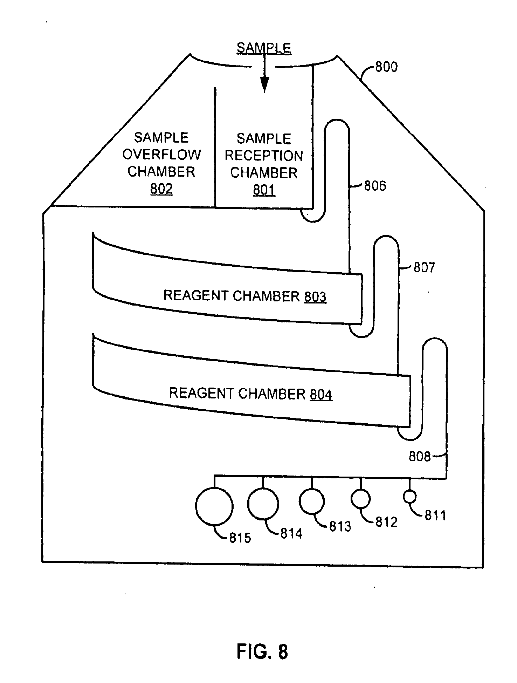

Figure 8 illustrates a titration rotor block 800 in an example of the invention. Rotor block 800 is typically comprised of clear plastic. Rotor block 800 includes sample reception chamber 801, sample overflow chamber 802, reagent chambers 803-804, titration chambers 811-815, and capillaries 806-808. Rotor block 800 would also include vents that are not shown for clarity. Rotor block 800 would be selected by the user and mounted on base 100 to facilitate a titration test on a sample of interest to the user.

In operation, the centrifugal force drives the sample into sample reception chamber 801. The combination of capillary action and centrifugal force transfer a precise amount of the sample from sample reception chamber 801 to reagent chamber 803 through capillary 806. Reagent chamber 803 contains a reagent that interacts with the sample. After the desired interaction, capillary action and centrifugal force transfer a precise amount of the reacted sample in reagent chamber 803 through capillary 807 to reagent chamber 804. Reagent chamber 804 also contains a reagent that interacts with the sample. After the desired interaction, capillary action and centrifugal force transfer precise amounts of the reacted sample in reagent chamber 804 through capillary 808 to titration chambers 811-815. In various alternatives, there may not be any reagent chambers (chamber 801 would directly feed chambers 811-815), one reagent chamber, or there may be more than two reagent chambers.

Titration chambers 811-815 each contain a titration reagent, so when the sample is loaded into titration chambers 811-815, titration chambers 811-815 each contain a different proportion of sample and titration reagent. In a titration, an event such as a color change is looked for to identify the respective proportion of sample and titration reagent that caused the event. Thus, the titration chamber that exhibits the event identifies this proportion. For example, the smallest chamber that changes color can indicate the proportion of interest.

To obtain the different proportions of sample and titration reagent in titration chambers 811-815, the same amount of a titration reagent could be loaded into titration chambers 811-815, and each titration chamber would receive a different amount of the processed sample, possibly based on the different sizes of titration chambers 811-815. Alternatively, different amounts of titration reagent could be placed in titration chambers 811-815, and each titration chamber would receive the same amount of the processed sample. The titration testing is described above with respect to a modular rotor block, but in some examples of the invention, the titration testing could also be implemented in an otherwise conventional disc-shaped rotor

### Method of Standard Additions Rotor Block

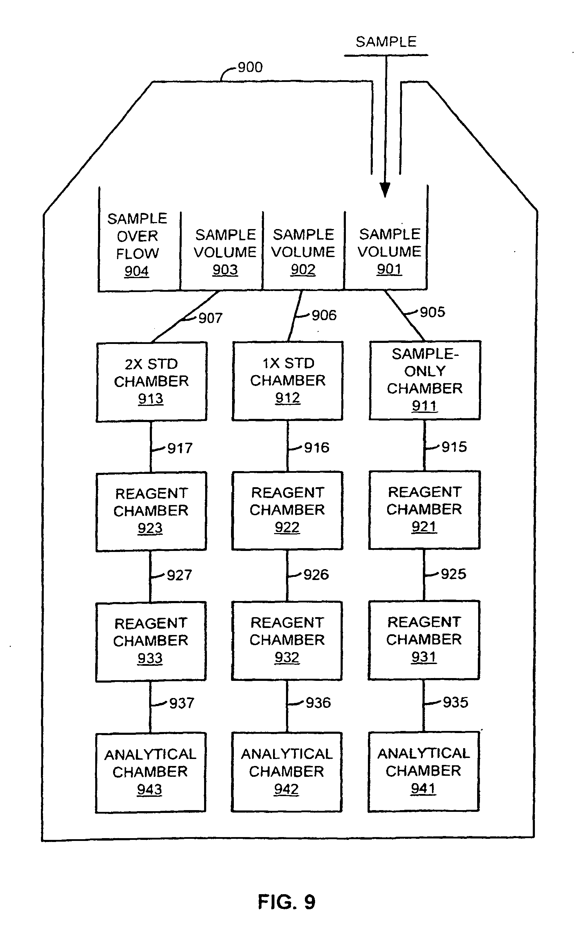

Figure 9 illustrates a Method of Standard Additions (MSA) rotor block 900 in an example of the invention. For clarity, figure 9 does not attempt to depict the physical characteristics of the chambers and capillaries as such are depicted for the examples described above. Rotor block 900 is typically comprised of clear plastic. Rotor block 900 includes sample volumes 901-903, sample overflow 904, chambers 911-913, 921-923, 931-933, and 941-943, and capillaries 905-907, 915-917, 925-927, and 935-937. Rotor block 900 would also include vents that are not shown for clarity. Rotor block 900 would be selected by the user and mounted on base 100 to facilitate an MSA test on a sample of interest to the user.

In operation, the centrifugal force drives the sample from the sample chamber on base 100 (not shown) into sample volume 901. When sample volume 901 is full, sample overflows into sample volume 902. When sample volume 902 is full, sample overflows into sample volume 903. When sample volume 903 is full, sample overflows into sample overflow 904. Thus, sample volumes 901-903 each contain a precise amount of the sample as defined by the overflow mechanism.

The combination of capillary action and centrifugal force transfer a precise amount of the sample from sample volumes 901-903 to respective chambers 911-913 through respective capillaries 905-907. In some examples, capillaries 905-907 have a restricted size to prevent sample flow from sample volumes 901-903 until the spin speed reaches a relatively high threshold. Other capillary designs could also be used.

Chambers 912-913 are each pre-loaded with a standard. The standard is typically the analyte of interest. The user may add the standard to chambers 912-913, but alternatively, block 900 may be configured so chambers 912-913 are pre-loaded with the standard for the user. In this example, chamber 913 has twice the standard of chamber 912, and chamber 911 has no standard and only receives the sample. Thus, chamber 911 includes just the sample with some unknown concentration of this analyte. Chamber 912 also includes a portion of the same sample, but this portion of the sample is spiked by the standard to include a higher concentration of the analyte. Chamber 913 also includes a portion of the same sample, and this portion is spiked by the standard to include an even higher concentration of the analyte. Advantageously, the final results may be assessed in light of the standard additions to ensure quality, since a quality result should reflect the spiking that occurs in chambers. For example, quality test results should indicate that chamber 943 has the highest concentration, and chamber 941 has the lowest concentration.

After standard addition, centrifugal force and capillary action drive the sample from chambers 911-913 to respective reagent chambers 921-923 through respective capillaries 915-917. Reagent chambers 921-923 each contain a reagent to react with the sample. After the reaction, centrifugal force and capillary action drive the sample from chambers 921-923 to respective reagent chambers 931-933 through respective capillaries 925-927. Reagent chambers 931-933 each contain a reagent to react with the sample. After the reaction, centrifugal force and capillary action drive the sample from chambers 931-933 to respective analytical chambers 941-943 through respective capillaries 935-937.

In some examples, analytical chambers 941-943 are vertically stacked in the manner of chambers 305 and 505 on figure 5, except that there are three stacked chambers in this example as opposed to two stacked chambers in figure 5. The stacked chambers 941-943 provide the beneficial longer analytical signal paths described with respect to figure 5. Alternatively, analytical chambers could be on the same plane and separated in a radial fashion near the edge of block 900.

Analytical device 110 (not shown) transfers analytical signals through respective analytical chambers 941-943, and then receives and processes the analytical signals to determine the concentration of the analyte in the sample. The results should reflect the standard additions, and if they do, the test is validated, and the concentration of the analyte in the sample within chamber 941 (no standard addition) can be trusted with confidence.

Note that this example may also be varied. There could be one or many process paths that perform standard additions. There could be more stages that add standard. There could be no reagent stages, one reagent stage, or many reagent stages. The three process paths could be horizontally spread across the block or vertically stacked within the block. The three process paths may be separated on three separate blocks. For example, a first block could have no standard addition, a second block could have a 1x standard addition, and a third block could have a 2x standard addition. All three blocks could be mounted on the same base to perform the test at the same time with a shared central sample chamber on the base. Those skilled in the art will appreciate other variations.

In addition, the same general block design could be used to carry out a spike-recovery assay. The MSA testing is described above with respect to a modular rotor block, but in some examples of the invention, the MSA testing could also be implemented in an otherwise conventional disc-shaped rotor.

### Filtration Rotor Block

A rotor block could perform filtration. The filtration could be performed by allowing centrifugal force to separate a substance in a chamber, and by providing an orifice or capillary at the point in the chamber that has the filtered portion of the substance. For example, a water sample could be introduced into a chamber, and centrifugal force could drive sediment in the water to the end of the chamber away from the center of the block. The water near the other end of the chamber toward the center of the block would then be sediment- free, and a capillary or orifice near this point could receive the filtered water. Alternatively, a filtration membrane could be placed across a chamber, so that centrifugal force would drive the substance through the membrane to filter the substance. For example, a membrane with pores of a given diameter could be used to filter particles from a sample that are larger than the pores. The sample filtration is described above with respect to a modular rotor block, but in some examples of the invention, the sample filtration could also be implemented in an otherwise conventional disc-shaped rotor

## Claims

1. An analytical rotor system (120) to perform a plurality of tests selected by a user on at least one sample, the analytical rotor system (120) comprising:
a plurality of rotor blocks (101-104) that are each configured to perform at least one of the tests on the at least one sample in response to centrifugal force, wherein the rotor blocks (101-104) are physically separate units from one another; and
a rotor base (100) that is a physically separate unit from the rotor blocks (101-104) and that is configured to allow the user to manually install the rotor blocks (101-104) on the rotor base (100), wherein the rotor base (100) is configured to hold the installed rotor blocks (101-104) in place during the centrifugal force and to connect to an analytical device (110) that provides the centrifugal force, **characterised in that** the rotor base (100) includes a sample chamber (105) configured to receive and hold the sample, and in response to the centrifugal force, to transfer the sample through a sample port (106) to one of the rotor blocks (101-104).

2. The analytical rotor system (120) of claim 1 wherein the rotor base (100) includes flanges (114) configured to secure the rotor blocks (101-104) to the rotor base (100) during the centrifugal force.

3. The analytical rotor system (120) of claim 1 wherein one of the tests is to determine a concentration of an analyte in the at least one sample and wherein the at least one sample comprises a water sample.

4. The analytical rotor system (120) of claim 1 wherein the sample chamber (105) is tapered so a fluid level of the sample does not reach the sample port (106) while the sample chamber (105) is at rest, but the fluid level of the sample does reach the sample port (106) when the sample chamber (105) is spinning.

5. The analytical rotor system (120) of claim 1 wherein one of the rotor blocks (101-104) is configured to filter the at least one sample.

6. The analytical rotor system (120) of claim 1 wherein one of the tests comprises a titration test.

7. The analytical rotor system (120) of claim 1 wherein at least one of the rotor blocks (101-104) includes a plurality of titration chambers (811-815) that hold different proportions of a titration reagent and the sample in response to the centrifugal force and wherein at least one of the titration chambers (811-815) indicates an event that corresponds to one of the proportions of the sample and the titration reagent.

8. The analytical rotor system (120) of claim 1 wherein one of the tests comprises a method of standard additions test.

9. The analytical rotor system (120) of claim 1 wherein at least one of the rotor blocks (101-104) includes a sample-only chamber (911) and a standard addition chamber (912), wherein the sample only chamber (911) is configured to hold a first portion of the sample in response to the centrifugal force but does not hold a standard, and wherein the standard addition chamber (912) is configured to hold a first portion of the standard that is added to a second portion of the sample in response to the centrifugal force.

10. The analytical rotor system (120) of claim 1 wherein one of the rotor blocks (101-104) includes an analytical chamber (305) that is configured to allow an analytical signal to traverse an analytical signal path through the analytical chamber (305) to perform one of the tests, wherein the analytical signal path is parallel to a plane of a spin that provides the centrifugal force.

11. A method of operating an analytical rotor system (120) to perform a plurality of tests selected by a user on at least one sample, the method comprising:
holding the at least one sample in a sample chamber (105) in a rotor base (100);
manually installing a plurality of rotor blocks (101-104) on the rotor base (100), wherein the rotor blocks (101-104) are physically separate units from one another, and wherein the rotor base (100) is a physically separate unit from the rotor blocks (101-104);
connecting the rotor base (100) to an analytical device (110); and
operating the analytical device (110) to spin the rotor base (100) to provide centrifugal force that causes each of the rotor blocks (101-104) to perform at least one of the tests on the at least one sample, **characterised in that** the rotor base (100) is configured to hold the installed rotor blocks (101-104) in place during the centrifugal force, and wherein operating the analytical device (110) to spin the rotor base (100) to provide the centrifugal force comprises transferring the sample from the sample chamber (105) through a sample port (106) to one of the rotor blocks (101-104) in response to the centrifugal force.

12. The method of claim 11 wherein the rotor base (100) includes flanges (114) configured to secure the rotor blocks (101-104) to the rotor base (100) during the centrifugal force.

13. The method of claim 11 wherein one of the tests is to determine a concentration of an analyte in the at least one sample and wherein the at least one sample comprises a water sample.

14. The method of claim 11 wherein the sample chamber (105) is tapered so a fluid level of the sample does not reach the sample port (106) while the sample chamber (105) is at rest, but the fluid level of the sample does reach the sample port (106) when the sample chamber (105) is spinning.

15. The method of claim 11 wherein one of the rotor blocks (101-104) filters the at least one sample to perform one of the tests.

16. The method of claim 11 wherein one of the tests comprises a titration test.

17. The method of claim 11 wherein at least one of the rotor blocks (101-104) includes a plurality of titration chambers (811-815) that hold different proportions of a titration reagent and the sample in response to the centrifugal force, and wherein to perform the at least one test, at least one of the titration chambers (811-815) indicates an event that corresponds to one of the proportions of the sample and the titration reagent.

18. The method of claim 11 wherein one of the tests comprises a method of standard additions test.

19. The method of claim 11 wherein at least one of the rotor blocks (101-104) includes a sample-only chamber (911) and a standard addition chamber (912), and wherein to perform the at least one test, the sample only chamber (911) holds a first portion of the sample in response to the centrifugal force but does not hold a standard, and wherein the standard addition chamber (912) holds a first portion of the standard that is added to a second portion of the sample in response to the centrifugal force.

20. The method of claim 11 wherein one of the rotor blocks (101-104) includes an analytical chamber (305) that is configured to allow an analytical signal to traverse an analytical signal path through the analytical chamber (305) to perform one of the tests, wherein the analytical signal path is parallel to a plane of a spin that provides the centrifugal force.

## Patentansprüche

1. Analytisches Rotorsystem (120) zur Durchführung einer Vielzahl von Prüfungen, die für mindestens eine Probe durch einen Nutzer ausgewählt werden, wobei das analytische Rotorsystem (120) umfasst:
eine Vielzahl von Rotorblöcken (101-104), von denen jeder konfiguriert ist, mindestens eine der Prüfungen an der mindestens einen Probe in Reaktion auf Zentrifugalkraft durchzuführen, wobei die Rotorblöcke (101-104) voneinander körperlich getrennte Einheiten sind; und
eine Rotorbasis (100), die eine von den Rotorblöcken (101-104) körperlich getrennte Einheit ist und die so konfiguriert ist, dass diese dem Nutzer ermöglicht, die Rotorblöcke (101-104) manuell auf der Rotorbasis (100) zu installieren, wobei die Rotorbasis (100) konfiguriert ist, die installierten Rotorblöcke (101-104) während der Zentrifugalkraft an Ort und Stelle zu halten und sich mit einer analytischen Einrichtung (110) zu verbinden, die die Zentrifugalkraft bereitstellt,
**dadurch gekennzeichnet, dass**
die Rotorbasis (110) eine Probenkammer (105) einschließt, die konfiguriert ist, die Probe aufzunehmen und zu halten und in Reaktion auf die Zentrifugalkraft die Probe durch einen Probenanschluss (106) zu einem der Rotorblöcke (101-104) zu übertragen.

2. Das analytische Rotorsystem (120) gemäß Anspruch 1, wobei die Rotorbasis (100) Flansche (114) einschließt, die konfiguriert sind, die Rotorblöcke (101-104) an der Rotorbasis (100) während der Zentrifugalkraft zu sichern.

3. Das analytische Rotorsystem (120) gemäß Anspruch 1, wobei eine der Prüfungen darin besteht, eine Konzentration eines Analyten in der mindestens einen Probe zu bestimmen und wobei die mindestens eine Probe eine Wasserprobe umfasst.

4. Das analytische Rotorsystem (120) gemäß Anspruch 1, wobei die Probenkammer (105) angeschrägt ist, so dass ein Fluidniveau der Probe den Probenanschluss (106) nicht erreicht, während die Probenkammer in Ruhe ist, das Fluidniveau der Probe aber den Probenanschluss (106) erreicht, wenn die Probenkammer (105) in Umdrehung ist.

5. Das analytische Rotorsystem (120) gemäß Anspruch 1, wobei einer der Rotorblöcke (101 - 104) konfiguriert ist, die mindestens eine Probe zu filtern.

6. Das analytische Rotorsystem (120) gemäß Anspruch 1, wobei eine der Prüfungen eine Titrationsprüfung umfasst.

7. Das analytische Rotorsystem (120) gemäß Anspruch 1, wobei mindestens einer der Rotorblöcke (101-104) eine Vielzahl von Titrationskammern (811-815) einschließt, die unterschiedliche Anteile eines Titrationsreagens und der Probe in Reaktion auf die Zentrifugalkraft halten, und wobei mindestens eine der Titrationskammern (811-815) ein Ereignis anzeigt, das einem der Anteile der Probe und des Titrationsreagens entspricht.

8. Das analytische Rotorsystem (120) gemäß Anspruch 1, wobei eine der Prüfungen ein Verfahren der Standardadditionsprüfung umfasst.

9. Das analytische Rotorsystem (120) gemäß Anspruch 1, wobei mindestens einer der Rotorblöcke (101-104) eine Kammer (911) ausschließlich für die Probe und eine Standardadditionskammer (912) einschließt, wobei die Kammer (911) ausschließlich für die Probe konfiguriert ist, einen ersten Anteil der Probe in Reaktion auf die Zentrifugalkraft zu halten, aber einen Standard nicht hält, und wobei die Standardadditionskammer (912) konfiguriert ist, einen ersten Anteil des Standards zu halten, der zu einem zweiten Anteil der Probe in Reaktion auf die Zentrifugalkraft addiert wird.

10. Das analytische Rotorsystem (120) gemäß Anspruch 1, wobei einer der Rotorblöcke (101-104) eine analytische Kammer (305) einschließt, die so konfiguriert ist, dass ein analytisches Signal einen analytischen Signalweg durch die analytischen Kammer (305) durchlaufen kann, um eine der Prüfungen durchzuführen, wobei der analytische Signalweg parallel zu einer Ebene einer Umdrehung ist, die die Zentrifugalkraft bereitstellt,

11. Ein Verfahren zum Betrieb eines analytischen Rotorsystems (120) zur Durchführung einer Vielzahl von Prüfungen, die durch einen Nutzer für mindestens eine Probe ausgewählt werden, wobei das Verfahren umfasst:
Halten der mindestens einen Probe in einer Probenkammer (105) in einer Rotorbasis (100);
manuelles installieren einer Vielzahl von Rotorblöcken (101-104) auf der Rotorbasis (100), wobei die Rotorblöcke (101-104) körperlich voneinander getrennte Einheiten sind und wobei die Rotorbasis (100) eine von den Rotorblöcken (101 - 104) körperlich getrennte Einheit ist;
Verbinden der Rotorbasis (100) mit einer analytischen Einrichtung (110);
Betreiben der analytischen Einrichtung (110), um die Rotorbasis (100) in Umdrehung zu versetzen, um eine Zentrifugalkraft bereitzustellen, die verursacht, dass jeder der Rotorblöcke (101-104) mindestens eine der Prüfungen an der mindestens einen Probe durchführt,
**dadurch gekennzeichnet, dass**
die Rotorbasis (100) konfiguriert ist, die installierten Rotorblöcke (101-104) während der Zentrifugalkraft an Ort und Stelle zu halten, und wobei der Betrieb der analytischen Einrichtung (110) um die Rotorbasis (100) im Umdrehung zu versetzen, um die Zentrifugalkraft bereitzustellen, die Übertragung der Probe von der Probenkammer (105) durch einen Probenanschluss (106) zu einem der Rotorblöcke (101-104) Reaktion auf die Zentrifugalkraft umfasst.

12. Das Verfahren gemäß Anspruch 11, wobei die Rotorbasis (100) Flansche (114) einschließt, die konfiguriert sind, die Rotorblöcke (101-104) an der Rotorbasis (100) während der Zentrifugalkraft zu sichern.

13. Das Verfahren gemäß Anspruch 11, wobei eine der Prüfungen darin besteht, eine Konzentration eines Analyten in der einen Probe zu bestimmen und wobei die mindestens eine Probe eine Wasserprobe umfasst.

14. Das Verfahren gemäß Anspruch 11, wobei die Probenkammer (105) angeschrägt ist, so dass ein Fluidniveau der Probe den Probenanschluss (106) nicht erreicht, während die Probenkammer in Ruhe ist, das Fluidniveau der Probe aber den Probenanschluss (106) erreicht, wenn die Probenkammer (105) in Umdrehung ist.

15. Das Verfahren gemäß Anspruch 11. wobei einer der Rotorblöcke (101-104), die mindestens eine Probe filtert, um eine der Prüfungen durchzuführen.

16. Das Verfahren gemäß Anspruch 11, wobei eine der Prüfungen eine Titrationsprüfung umfasst,

17. Das Verfahren gemäß Anspruch 11, wobei mindestens einer der Rotorblöcke (101-104) eine Vielzahl von Titrationskammern (811-815) einschließt, die unterschiedliche Anteile eines Titrationsreagens und der Probe in Reaktion auf die Zentrifugalkraft halten, und wobei, um die mindestens eine Prüfung durchzuführen, mindestens eine der Titrationskammern (811-815) ein Ereignis anzeigt, das einem der Anteile der Probe und des Titrationsreagens entspricht.

18. Das Verfahren gemäß Anspruch 11, wobei eine der Prüfungen ein Verfahren der Standardadditionsprüfung umfasst.

19. Das Verfahren gemäß Anspruch 11, wobei mindestens einer der Rotorblöcke (101-104) eine Kammer (911) ausschließlich für die Probe und eine Standardadditionskammer (912) einschließt, und wobei, um die mindestens eine Prüfung durchzuführen, die Kammer (911) ausschließlich für die Probe einen ersten Anteil der Probe in Reaktion auf die Zentrifugalkraft hält, aber einen Standard nicht hält, und wobei die Standardadditionskammer (912) einen ersten Anteil des Standards hält, der zu einem zweiten Anteil der Probe in Reaktion auf die Zentrifugalkraft addiert wird.

20. Das Verfahren gemäß Anspruch 11, wobei einer der Rotorblöcke (101-104) eine analytische Kammer (305) einschließt, die so konfiguriert ist, dass ein analytisches Signal einen analytischen Signalweg durch die analytischen Kammer (305) durchlaufen kann, um eine der Prüfungen durchzuführen, wobei der analytische Signalweg parallel zu einer Ebene einer Umdrehung ist, die die Zentrifugalkraft bereitstellt.

## Revendications

1. Système à rotor analytique (120) pour réaliser une pluralité d'essais sélectionnés par un utilisateur sur au moins un échantillon, le système à rotor analytique (120) comprenant :
une pluralité de blocs de rotor (101-104) qui sont chacun configurés pour réaliser au moins l'un des essais sur le au moins un échantillon en réponse à la force centrifuge, dans lequel les blocs de rotor (101-104) sont des unités physiquement séparées les unes des autres ; et
une base de rotor (100) qui est une unité physiquement séparée des blocs de rotor (101-104) et qui est configurée pour permettre à l'utilisateur d'installer manuellement les blocs de rotor (101-104) sur la base de rotor (100), dans lequel la base de rotor (100) est configurée pour maintenir les blocs de rotor (101-104) installés, en place pendant la force centrifuge et pour le raccorder à un dispositif analytique (110) qui fournit la force centrifuge, **caractérisé en ce que** la base de rotor (100) comprend une chambre d'échantillon (105) configurée pour recevoir et contenir l'échantillon et en réponse à la force centrifuge, transférer l'échantillon par un orifice d'échantillon (106) vers l'un des blocs de rotor (101-104).

2. Système à rotor analytique (120) selon la revendication 1, dans lequel la base de rotor (100) comprend des rebords (114) configurés pour fixer les blocs de rotor (101-104) sur la base de rotor (100) pendant la force centrifuge.

3. Système à rotor analytique (120) selon la revendication 1, dans lequel l'un des essais consiste à déterminer une concentration d'une substance à analyser dans le au moins un échantillon et dans lequel le au moins un échantillon comprend un échantillon d'eau.

4. Système à rotor analytique (120) selon la revendication 1, dans lequel la chambre d'échantillon (105) est progressivement rétrécie de sorte qu'un niveau de fluide de l'échantillon n'atteint pas l'orifice d'échantillon (106) alors que la chambre d'échantillon (105) est au repos, mais le niveau de fluide de l'échantillon atteint l'orifice d'échantillon (106) lorsque la chambre d'échantillon (105) tourne.

5. Système à rotor analytique (120) selon la revendication 1, dans lequel l'un des blocs de rotor (101-104) est configuré pour filtrer le au moins un échantillon.

6. Système à rotor analytique (120) selon la revendication 1, dans lequel l'un des essais comprend un essai de titrage.

7. Système à rotor analytique (120) selon la revendication 1, dans lequel au moins l'un des blocs de rotor (101-104) comprend une pluralité de chambres de titrage (811-815) qui contiennent différentes proportions d'un réactif de titrage et de l'échantillon en réponse à la force centrifuge et dans lequel au moins l'une des chambres de titrage (811-815) indique un cas qui correspond à l'une des proportions de l'échantillon et du réactif de titrage.

8. Système à rotor analytique (120) selon la revendication 1, dans lequel l'un des essais comprend un procédé d'essai de dosage par ajouts dosés d'échantillon standard.

9. Système à rotor analytique (120) selon la revendication 1, dans lequel au moins l'un des blocs de rotor (101-104) comprend une chambre ne contenant que l'échantillon (911) et une chambre d'échantillon standard (912) dans lequel la chambre ne contenant que l'échantillon (911) est configurée pour contenir une première partie de l'échantillon en réponse à la force centrifuge mais ne contient pas un échantillon standard, et dans lequel la chambre de dosage par ajouts dosés d'échantillon standard (912) est configurée pour contenir une première partie de l'échantillon standard qui est ajoutée à une seconde partie de l'échantillon en réponse à la force centrifuge.

10. Système à rotor analytique (120) selon la revendication 1, dans lequel l'un des blocs de rotor (101-104) comprend une chambre analytique (305) qui est configurée pour permettre à un signal analytique de traverser un chemin de signal analytique en passant par la chambre analytique (305) pour réaliser l'un des essais, dans lequel le chemin de signal analytique est parallèle à un plan d'une vrille qui fournit la force centrifuge.

11. Procédé de fonctionnement d'un système à rotor analytique (120) pour réaliser une pluralité d'essais sélectionnés par un utilisateur sur au moins un échantillon, le procédé comprenant les étapes consistant à :
contenir le au moins un échantillon dans une chambre d'échantillon (105) dans une base de rotor (100) ;
installer manuellement une pluralité de blocs de rotor (101-104) sur la base de rotor (100),
dans lequel les blocs de rotor (101-104) sont des unités physiquement séparées les unes des autres, et
dans lequel la base de rotor (100) est une unité physiquement séparée des blocs de rotor (101-104) ;
raccorder la base de rotor (100) à un dispositif analytique (110) ; et
actionner le dispositif analytique (110) pour faire tourner la base de rotor (100) pour fournir la force centrifuge qui amène chacun des blocs de rotor (101-104) à réaliser au moins l'un des essais sur le au moins un échantillon, **caractérisé en ce que** la base de rotor (100) est configurée pour maintenir les blocs de rotor (101-104) installés en place pendant la force centrifuge, et dans lequel l'étape consistant à actionner le dispositif analytique (110) pour faire tourner la base de rotor (100) afin de fournir la force centrifuge comprend l'étape consistant à transférer l'échantillon de la chambre d'échantillon (105) en passant par un orifice d'échantillon (106) vers l'un des blocs de rotor (101-104) en réponse à la force centrifuge.

12. Procédé selon la revendication 11, dans lequel la base de rotor (100) comprend des rebords (114) configurés pour fixer les blocs de rotor (101-104) sur la base de rotor (100) pendant la force centrifuge.

13. Procédé selon la revendication 11, dans lequel l'un des essais consiste à déterminer une concentration d'une substance à analyser dans le au moins un échantillon et dans lequel le au moins un échantillon comprend un échantillon d'eau.

14. Procédé selon la revendication 11, dans lequel la chambre d'échantillon (105) est progressivement rétrécie de sorte qu'un niveau de fluide de l'échantillon n'atteint pas l'orifice d'échantillon (106) alors que la chambre d'échantillon (105) est au repos, mais le niveau de fluide de l'échantillon n'atteint pas l'orifice d'échantillon (106) lorsque la chambre d'échantillon (105) tourna.

15. Procédé selon la revendication 11, dans lequel l'un des blocs de rotor (101-104) filtre le au moins un échantillon pour réaliser l'un des essais.

16. Procédé selon la revendication 11, dans lequel l'un des essais comprend un essai de titrage.

17. Procédé selon la revendication 11, dans lequel au moins l'un des blocs de rotor (101-104) comprend une pluralité de chambres de titrage (811-815) qui contiennent différentes proportions de réactif de titrage et d'échantillon en réponse à la force centrifuge, dans lequel afin de réaliser le au moins un essai, au moins l'une des chambres de titrage (811-815) indique un cas qui correspond à l'une des proportions de l'échantillon et du réactif de titrage.

18. Procédé selon la revendication 11, dans lequel l'un des essais comprend un procédé de dosage par ajouts dosés d'échantillon standard.

19. Procédé selon la revendication 11, dans lequel au moins l'un des blocs de rotor (101-104) comprend une chambre ne contenant que l'échantillon (911) et une chambre de dosage par ajouts dosés d'échantillon standard (912) et dans lequel pour réaliser le au moins un essai, la chambre ne contenant que l'échantillon (911) contient une première partie de l'échantillon en réponse à la force centrifuge mais ne contient pas un échantillon standard, et dans lequel la chambre de dosage par ajouts dosés d'échantillon standard (912) contient une première partie de l'échantillon standard qui est ajoutée à une seconde partie de l'échantillon en réponse à la force centrifuge.

20. Procédé selon la revendication 11, dans lequel l'un des blocs de rotor (101-104) comprend une chambre analytique (305) qui est configurée pour permettre à un signal analytique de traverser un chemin de signal analytique en passant par la chambre analytique (305) pour réaliser l'un des essais, dans lequel le chemin de signal analytique est parallèle à un plan d'une vrille qui fournit la force centrifuge.
